# EUROPÄISCHE PATENTSCHRIFT

(11) **EP 0 819 670 B1**
(45) Veröffentlichungstag und Bekanntmachung des Hinweises auf die Patenterteilung: **05.04.2000**
(21) Anmeldenummer: 97111720.5
(22) Anmeldetag: 10.07.1997
(51) Int. Cl.: C07C 59/01, C07C 51/235

(54) **Verfahren zur Herstellung von 3-Hydroxypropionsäure oder einem Salz derselben**
Process for preparing 3-hydroxypropionic acid or its salt
Procédé de préparation d'acide 3-hydroxypropionique ou son sel

(30) Priorität: 20.07.1996 DE 19629371
(43) Veröffentlichungstag der Anmeldung: 21.01.1998
(73) Patentinhaber: Degussa-Hüls Aktiengesellschaft, 60287 Frankfurt am Main (DE)
(72) Erfinder: Haas, Thomas, Dr., 60316 Frankfurt (DE); Brossmer, Christoph, Dr., 60318 Frankfurt (DE); Meier, Martin, Dr., 59457 Werl (DE); Arntz, Dietrich, Dr., 63829 Oberursel (DE); Freund, Andreas, Dr., 63801 Kleinostheim (DE)

(56) Entgegenhaltungen:
- WO-A-92/16489
- BE-A- 664 718
- GB-A- 573 334

## Beschreibung

Die Erfindung richtet sich auf ein Verfahren zur Herstellung von 3-Hydroxypropionsäure oder einem Salz derselben, das auf der katalytischen Oxidation eines C₃-Bausteins mit Sauerstoff oder einem sauerstoffhaltigen Gas in Gegenwart eines Edelmetallkatalysators basiert. Erfindungsgemäß wird 3-Hydroxypropionaldehyd als C₃-Baustein verwendet.

3-Hydroxypropionsäure sowie deren wasserlösliche Salze sind wertvolle Synthesebausteine. 3-Hydroxypropionsäure wird üblicherweise durch Wasseranlagerung an Acrylsäure oder durch Umsetzung von Ethylenchlorhydrin mit Natriumcyanid hergestellt (Ullman's Encyclopedia of Industrial Chemistry, 5. Auflage, Band A-13, Seiten 507-517). Im Falle der Acrylsäurehydratisierung handelt es sich um eine Gleichgewichtsreaktion, d.h. die Umsätze sind limitiert. Im Falle der Umsetzung des Ethylenchlorhydrins ist der Einsatz hochtoxischer Stoffe erforderlich und zudem muß noch eine Hydrolysestufe angeschlossen werden. Dabei kommt es zu einem hohen Salzanfall von Natriumchlorid und Ammoniumsalzen. Diese Nachteile lassen sich gemäß DE-A-4 107 987 vermeiden, indem 1,3-Propandiol in Gegenwart eines Palladium enthaltenden Trägerkatalysators mit Sauerstoff oder einem sauerstoffhaltigen Gas zu 3-Hydroxypropionsäure oxidiert wird. Ausweislich der Beispiele wird 3-Hydroxypropionsäure in einer Ausbeute bis zu etwa 82 % erhalten. Gemäß JP-A 56/5433 ist durch katalytische Oxidation von 1,3-Propandiol in Gegenwart von 3,3 Gew.-% Palladium, bezogen auf das Diol, 3-Hydroxypropionsäure in einer Ausbeute von 84 % erhältlich. Dieses Dokument lehrt aber nicht, die Oxidation nur bis zur Stufe der 3-Hydroxypropionsäure zu betreiben.

Gemäß EP-B 0 350 741 läßt sich Glucose, also ein Hydroxyaldehyd, in Gegenwart eines Pd- oder Pt-Katalysators zu Gluconsäure oxidieren. Mit den in diesem Dokument angegebenen Katalysatormengen von unter 2 Gew.-% Edelmetall, bezogen auf Glucose, läßt sich, wie festgestellt wurde, 3-Hydroxypropionaldehyd nicht in höherer Ausbeute zu 3-Hydroxypropionsäure oxidieren.

Die Aufgabe der vorliegenden Erfindung besteht darin, ein weiteres Verfahren zur Herstellung von 3-Hydroxypropionsäure oder einem Salz derselben durch katalytische Oxidation eines C₃-Bausteins aufzuzeigen, das zu einer hohen Ausbeute führt. Vorzugsweise sollte das Verfahren so durchgeführt werden können, daß 3-Hydroxypropionsäure aus einem salzfreien Reaktionsgemisch isoliert werden kann.

Gefunden wurde demgemäß ein Verfahren zur Herstellung von 3-Hydroxypropionsäure oder einem Salz derselben, umfassend katalytische Oxidation eines C₃-Bausteins mit Sauerstoff oder einem sauerstoffhaltigen Gas in Gegenwart eines ein Edelmetall aus der Platingruppe enthaltenden Katalysators in wäßriger Phase und Isolierung der 3-Hydroxypropionsäure oder eines Salzes derselben aus dem Reaktionsgemisch, das dadurch gekennzeichnet ist, daß man als C₃-Baustein 3-Hydroxypropionaldehyd verwendet und den Katalysator in einer Menge entsprechend mindestens 10 Gew.-% Edelmetall, bezogen auf den C₃-Baustein, einsetzt.

Die Unteransprüche richten sich auf bevorzugte Ausführungsformen des erfindungsgemäßen Verfahrens. C₃-Baustein im erfindungsgemäßen Verfahren ist 3-Hydroxypropionaldehyd. Dieser Rohstoff ist leichter zugänglich als das bisher verwendete 1,3-Propandiol, das fermentativ aus Glycerin oder durch Hydratisierung von Acrolein mit anschließender Hydrierung des Hydroxypropionaldehyds erhältlich ist (siehe z.B. EP-B 0 412 337). Durch die Verwendung von 3-Hydroxypropionaldehyd als C₃-Baustein im erfindungsgemäßen Verfahren wird demgemäß ein Oxidationsäquivalent weniger benötigt als zur Oxidation von 1,3-Propandiol.

Die katalytische Oxidation wird durch ein Edelmetall aus der Platingruppe, also Ru, Rh, Pd, Os, Ir und Pt, katalysiert. Bevorzugte Katalysatoren enthalten Palladium oder Platin. Es kann sich hierbei um reine Metallkatalysatoren handeln oder um Trägerkatalysatoren. Palladium und Platin enthaltende Trägerkatalysatoren werden bevorzugt; als Trägermaterial eignen sich insbesondere Aktivkohle sowie oxidische und silikatische Materialien.

Ein erfindungsgemäßes Merkmal ist die bei der katalytischen Oxidation einzusetzende Menge Katalysator: Es wurde nämlich festgestellt, daß durch Erhöhung der Katalysatormenge - die Bezugsgröße ist hierbei jeweils die Edelmetallmenge - die Ausbeute sprunghaft ansteigt und damit 3-Hydroxypropionsäure in nahezu quantitativer Ausbeute erhältlich ist. Üblicherweise wird der Katalysator in einer Menge entsprechend mindestens 10 Gew.-% Edelmetall, bezogen auf den C₃-Baustein, eingesetzt; bevorzugt wird eine Einsatzmenge von mindestens 20 Gew.-% Edelmetall, bezogen auf den C₃-Baustein.

Die katalytische Oxidation kann sowohl unter Verwendung eines Suspensionskatalysators in einem Suspensionsreaktor oder unter Verwendung eines Festbettkatalysators in einem Festbettreaktor durchgeführt werden. Soweit der Katalysator, vorzugsweise ein Trägerkatalysator, in einem Festbettreaktor angeordnet ist, kann dieser sowohl in der Rieselbettfahrweise als auch in der Sumpffahrweise betrieben werden. In der Rieselbettfahrweise können die wäßrige Phase, enthaltend den C₃-Baustein, sowie Oxidationsprodukte desselben und ein Mittel zur pH-Einstellung, und Sauerstoff oder ein sauerstoffhaltiges Gas im Gleich- oder Gegenstrom geführt werden. Bei der Sumpffahrweise werden die flüssige Phase und die Gasphase üblicherweise im Gleichstrom geführt. Während sich bei der Rieselbettfahrweise automatisch eine ausreichend große Austauschfläche zwischen der flüssigen Phase und der Gasphase ergibt, ist im Falle der Sumpffahrweise sowie bei der Oxidation in einem Suspensionsreaktor auf eine gute Dispergierung des Sauerstoffs oder sauerstoffhaltigen Gases in der flüssigen Phase zu achten.

Da erfindungsgemäß der Katalysator in einer möglichst großen Menge, bezogen auf den C₃-Baustein, eingesetzt werden soll, ist die Oxidation unter Verwendung eines Festbettreaktors besonders vorteilhaft. Bei dieser Ausführungsform wird das erforderliche Gewichtsverhältnis quasi automatisch erzielt. Das Reaktionsgemisch kann ein oder mehrmals über den Festbettreaktor geleitet werden, so daß außerhalb des Reaktors jeweils der pH-Wert angepaßt werden kann.

Der C₃-Baustein wird der Oxidationsreaktion in wäßriger Phase zugeführt. Üblicherweise liegt die Konzentration des C₃-Bausteins in der wäßrigen Phase im Bereich zwischen 1 und 30 Gew.-%, vorzugsweise zwischen 1 und 10 Gew.-%. 3-Hydroxypropionaldehyd als C₃-Baustein kann in Form einer wäßrigen Lösung mit einem Gehalt zwischen etwa 5 und 20 Gew.-%, wie sie durch sauer katalysierte Hydratisierung von Acrolein mit nachfolgender Abtrennung des nicht umgesetzten Acroleins leicht erhältlich ist, unmittelbar oder nach Verdünnung mit Wasser eingesetzt werden.

Um insbesondere bei Verwendung eines Palladiumkatalysators eine ausreichend kurze Reaktionszeit zu erzielen, wird die Umsetzung bei einem pH-Wert von gleich oder größer 6, vorzugsweise mindestens 7, und insbesondere zwischen 7,5 und 9, durchgeführt. Gemäß einer bevorzugten Ausführungsform wird während der Oxidationsreaktion der pH-Wert durch Zugabe einer Base konstant gehalten, vorzugsweise auf einem pH-Wert im Bereich zwischen 7,5 und 9. Als Base wird vorzugsweise eine 10 bis 50 gew.-%ige wäßrige Alkalimetall- oder Erdalkalimetallhydroxidlösung verwendet.

Gemäß einer besonders bevorzugten Ausführungsform wird ein Platinkatalysator, insbesondere ein Platin enthaltender Trägerkatalysator eingesetzt. Überraschenderweise ist hierbei eine pH-Regulierung nicht nötig. Damit erfolgt die Oxidation in saurer Phase und 3-Hydroxypropionsäure kann unmittelbar aus dem Reaktionsgemisch isoliert werden. Das Verfahren verläuft also salzfrei, wodurch der technische Aufwand gemindert werden konnte.

Die Oxidation wird zweckmäßigerweise bei einer Temperatur von mindestens 10 °C und maximal 70 °C durchgeführt. Ein bevorzugter Temperaturbereich liegt zwischen 20 und 60 °C, insbesondere zwischen 40 und 60 °C. Bei einer Temperatur oberhalb 70 °C ist 3-Hydroxypropionaldehyd wenig stabil und zudem kommt es zur Verfärbung.

Der Durchfluß an Sauerstoff ist nicht limitiert. Wichtig ist bei der Suspensionsfahrweise, die flüssige und gasförmige Phase durch Intensivrühren miteinander in Kontakt zu bringen. Die Umsetzung ist nicht sehr druckabhängig, so daß sie zweckmäßigerweise unter autogenem oder geringfügig erhöhten Druck, etwa bis 3 bar, durchgeführt wird.

Aus dem nach Beendigung der bei einem pH-Wert von größer 6, insbesondere größer 7, durchgeführten Oxidationsreaktion erhaltenen 3-Hydroxypropionsäure-Salz enthaltenden wäßrigen Reaktionsgemisch kann 3-Hydroxypropionsäure in an sich bekannter Weise gewonnen werden, beispielsweise durch Umsetzung mit Schwefelsäure und Extraktion der 3-Hydroxypropionsäure oder durch Behandlung der Reaktionslösung mit einem sauren Ionenaustauscher.

Wie aus den nachfolgenden Beispielen und Vergleichsbeispielen deutlich hervorgeht, ist es durch die Verwendung der anspruchsgemäßen Menge Katalysator möglich, 3-Hydroxypropionsäure in sehr hoher Ausbeute zu erhalten. Gemäß bevorzugter Ausführungsform entfällt die Aufarbeitung eines salzhaltigen Reaktionsgemischs.

### Beispiele

Die nachfolgenden Beispiele und Vergleichsbeispiele wurden alle in einem 1 l Rührkolben durchgeführt. Eingesetzt wurde stets eine wäßrige 3-Hydroxypropionaldehyd(=HPA)-Lösung. Es wurde über eine Fritte mit 30 l/h Sauerstoff begast. Die Reaktionstemperatur betrug 50 °C. Zur Einstellung des pH-Werts, soweit durchgeführt, wurde Natronlauge verwendet. Als Katalysator wurden in den Beispielen und Vergleichsbeispielen 3 % beziehungsweise 5 % Pd/Aktivkohle (E 105 XR/W 3 % beziehungsweise 5 % der Fa. Degussa AG) sowie 5 % Pt/Aktivkohle (F 105 R/W 5 % der Fa. Degussa AG) eingesetzt.

Die Produktzusammensetzung wurde mittels HPLC analysiert. Zur Trennung wurden zwei in Reihe geschaltete Aminosäulen SEPIL der Fa. Jasco verwendet. Als Eluent wurde eine Mischung aus Acetonitril und 0,03 molarer KH₂PO₄-Lösung im Verhältnis 6 zu 4 verwendet. Die Säulentemperatur betrug 35 °C, die Flußrate 1 ml/min.

### Vergleichsbeispiel 1

5 g Katalysator 5 % Pd/Aktivkohle, 90 g 10 %ige HPA-Lösung und 400 g Wasser wurden gemischt und in einem 1 l Rührkolben mit 30 l/h Sauerstoff begast. Die Reaktionstemperatur betrug 50 °C. Während der Reaktion wurde soviel Natronlauge zugeführt, daß der pH-Wert bei pH = 8 blieb. Nach 2,5 h Reaktionszeit wurde die Produktzusammensetzung mittels HPLC analysiert. Es wurde ein HPA-Umsatz von 83,5 % und eine Hydroxypropionsäureselektivität von 66 %, entsprechend einer Ausbeute von 55,1 % erzielt.

### Vergleichsbeispiel 2

5 g Katalysator 5 % Pd/Aktivkohle, 90 g 10 %ige HPA-Lösung und 400 g Wasser wurden gemischt und in einem 1 l Rührkolben mit 30 l/h Sauerstoff begast. Die Reaktionstemperatur betrug 50 °C. Auf eine pH-Regelung während der Reaktion wurde verzichtet. Nach 8,5 h Reaktionszeit wurde die Produktzusammensetzung mittels HPLC analysiert. Es wurde ein HPA-Umsatz von 4,8 % und eine Hydroxypropionsäureselektivität von 99,5 %, entsprechend einer Ausbeute von 4,8 % erzielt.

### Beispiel 1

100 g Katalysator 3 % Pd/Aktivkohle, 90 g 10 %ige HPA-Lösung und 500 g Wasser wurden gemischt und in einem 1 l Rührkolben mit 30 l/h Sauerstoff begast. Die Reaktionstemperatur betrug 50 °C. Während der Reaktion wurde soviel Natronlauge zugeführt, daß der pH-Wert bei pH = 8 blieb. Nach 1 h Reaktionszeit wurde die Produktzusammensetzung mittels HPLC analysiert. Es wurde ein HPA-Umsatz von 87,9 % und eine Hydroxypropionsäureselektivität von 89,5 %, entsprechend einer Ausbeute von 78,7 % erzielt.

Der Vergleich von Beispiel 1 mit dem Vergleichsbeispiel 1 zeigt, daß erst durch einen hohen Anteil an Katalysator im Reaktor eine befriedigende Hydroxypropionsäureselektivität und damit auch hohe Ausbeute erzielt wird. Außerdem wird durch diese Maßnahme die Reaktionszeit deutlich verkürzt. Aus den Vergleichsbeispielen 1 und 2 folgt, daß bei Verwendung eines Palladiumkatalysators eine pH-Regelung notwendig ist, da sonst kein ausreichender Umsatz erzielt werden kann.

### Vergleichsbeispiel 3

5,5 g Katalysator 5 % Pt/Aktivkohle, 90 g 10 %ige HPA-Lösung und 400 g Wasser wurden gemischt und in einem 1 l Rührkolben mit 30 l/h Sauerstoff begast. Die Reaktionstemperatur betrug 50 °C. Auf eine pH-Regelung während der Reaktion wurde verzichtet. Nach 4 h Reaktionszeit wurde die Produktzusammensetzung mittels HPLC analysiert. Es wurde ein HPA-Umsatz von 14,6 % und eine Hydroxypropionsäureselektivität von 98,5 %, entsprechend einer Ausbeute von 14,4 % erzielt.

### Beispiel 2

50 g Katalysator 5 % Pt/Aktivkohle, 90 g 10 %ige HPA-Lösung und 450 g Wasser wurden gemischt und in einem 1 l Rührkolben mit 30 l/h Sauerstoff begast. Die Reaktionstemperatur betrug 50 °C. Auf eine pH-Regelung während der Reaktion wurde verzichtet. Nach 2,5 h Reaktionszeit wurde die Produktzusammensetzung mittels HPLC analysiert. Es wurde ein HPA-Umsatz von 87,4 % und eine Hydroxypropionsäureselektivität von 93,6 %, entsprechend einer Ausbeute von 84,4 % erzielt.

### Beispiel 3

50 g Katalysator 5 % Pt/Aktivkohle, 90 g 10 %ige HPA-Lösung und 450 g Wasser wurden gemischt und in einem 1 l Rührkolben mit 30 l/h Sauerstoff begast. Die Reaktionstemperatur betrug 50 °C. Auf eine pH-Regelung während der Reaktion wurde verzichtet. Nach 4 h Reaktionszeit wurde die Produktzusammensetzung mittels HPLC analysiert. Es wurde ein HPA-Umsatz von 97,2 % und eine Hydroxypropionsäureselektivität von 95,6 %, entsprechend einer Ausbeute von 92,9 % erzielt.

Die Beispiele 2 und 3 zeigen, daß mit einem Pt-Katalysator eine pH-Regulierung nicht erforderlich ist.

## Patentansprüche

1. Verfahren zur Herstellung von 3-Hydroxypropionsäure oder einem Salz derselben, umfassend katalytische Oxidation eines C₃-Bausteins mit Sauerstoff oder einem sauerstoffhaltigen Gas in Gegenwart eines ein Edelmetall aus der Platingruppe enthaltenden Katalysators in wäßriger Phase und Isolierung der 3-Hydroxypropionsäure oder einem Salz derselben aus dem Reaktionsgemisch,
dadurch gekennzeichnet,
daß man als C₃-Baustein 3-Hydroxypropionaldehyd verwendet und den Katalysator in einer Menge entsprechend mindestens 10 Gew.-% Edelmetall, bezogen auf den C₃-Baustein, einsetzt.

2. Verfahren nach Anspruch 1,
dadurch gekennzeichnet,
daß man den Katalysator in einer Menge entsprechend 20 bis 50 Gew.-%, bezogen auf den C₃-Baustein, einsetzt.

3. Verfahren nach Anspruch 1 oder 2,
dadurch gekennzeichnet,
daß man als Katalysator einen Palladium oder Platin enthaltenden Trägerkatalysator verwendet.

4. Verfahren nach Anspruch 3,
dadurch gekennzeichnet,
daß man den Katalysator in einem Festbettreaktor anordnet und diesen
(i) in Rieselbettfahrweise, wobei die wäßrige Phase und Sauerstoff oder ein sauerstoffhaltiges Gas im Gleich- oder Gegenstrom geführt werden, oder
(ii) in Sumpffahrweise mit Gleichstromführung der flüssigen Phase und des Gases betreibt.

5. Verfahren nach einem der Ansprüche 1 bis 4,
dadurch gekennzeichnet,
daß man die Oxidation unter Verwendung eines Palladium enthaltenden Katalysators bei einem pH-Wert von mindestens 6, vorzugsweise einem pH-Wert zwischen 7,0 und 9, durchführt.

6. Verfahren nach einem der Ansprüche 1 bis 5,
dadurch gekennzeichnet,
daß man die Oxidation bei einer Temperatur im Bereich von 20 bis 60 °C durchführt.

7. Verfahren nach einem der Ansprüche 1 bis 4,
dadurch gekennzeichnet,
daß man die katalytische Oxidation unter Verwendung eines Platin enthaltenden Trägerkatalysators ohne pH-Regulierung durchführt.

## Claims

1. A process for preparing 3-hydroxypropionic acid or a salt of same, comprising catalytic oxidation of a C₃ unit with oxygen or an oxygen-containing gas, in the presence of a catalyst which contains a noble metal from the platinum group, in aqueous phase and isolation of the 3-hydroxypropionic acid or a salt of same from the reaction mixture,
characterised in that
3-hydroxypropionaldehyde is used as a C₃ unit and the catalyst is used in an amount corresponding to at least 10 wt.% of noble metal, with respect to the C₃ unit.

2. A process according to claim 1,
characterised in that
the catalyst is used in an amount corresponding to 20 to 50 wt.%, with respect to the C₃ unit.

3. A process according to claim 1 or 2,
characterised in that
a supported catalyst which contains palladium or platinum is used as catalyst.

4. A process according to claim 3,
characterised in that
the catalyst is located in a fixed bed reactor and this is operated
(i) in trickle bed mode, wherein the aqueous phase and oxygen or an oxygen-containing gas are passed through it as cocurrents or countercurrents, or
ii) in distillation mode with the liquid phase and the gas being passed through as cocurrents.

5. A process according to one of claims 1 to 4,
characterised in that
oxidation is performed using a catalyst which contains palladium at a pH of at least 6, preferably at a pH between 7.0 and 9.

6. A process according to one of claims 1 to 5,
characterised in that
oxidation is performed at a temperature in the range 20 to 60°C.

7. A process according to one of claims 1 to 4,
characterised in that
catalytic oxidation using a supported catalyst which contains platinum is performed without controlling the pH.

## Revendications

1. Procédé de préparation d'acide 3-hydroxypropionique ou d'un sel de celui-ci, comprenant l'oxydation catalytique d'un composant en C₃ avec de l'oxygène ou un gaz contenant de l'oxygène, en présence d'un catalyseur contenant un métal noble issu du groupe du platine, en phase aqueuse, et isolation de l'acide 3-hydroxypropionique, ou d'un sel de celui-ci, depuis le mélange de réaction,
caractérisé en ce qu'
on utilise comme composant en C₃ l'aldéhyde 3-hydroxypropionique et le catalyseur en une quantité correspondant au moins à 10 % en poids de métal noble, en se référant au composant en C₃.

2. Procédé selon la revendication 1,
caractérisé en ce qu'
on utilise le catalyseur en une quantité correspondant à 20 à 50 % en poids en se référant au composant en C₃.

3. Procédé selon la revendication 1 ou 2,
caractérisé en ce qu'
on utilise comme catalyseur un catalyseur porteur contenant du palladium ou du platine.

4. Procédé selon la revendication 3,
caractérisé en ce qu'
on dispose le catalyseur dans un réacteur à lit fixe et on conduit celui-ci
(i) dans un mode à passage en lit fluidisé, la phase aqueuse et l'oxygène, ou un gaz contenant de l'oxygène, étant guidés en co-courant ou en contre-courant, ou
(ii) on fonctionne en un mode à distillation poussée ou bas de colonne, avec un guidage en co-courant de la phase liquide et du gaz.

5. Procédé selon l'une des revendications 1 à 4, caractérisé en ce qu'on effectue l'oxydation en utilisant un catalyseur contenant du palladium, à une valeur de pH d'au moins 6, de préférence une valeur de pH comprise entre 7,0 et 9.

6. Procédé selon l'une des revendications 1 à 5,
caractérisé en ce qu'
on effectue l'oxydation à une température dans la plage de 20 à 60°C.

7. Procédé selon l'une des revendications 1 à 4,
caractérisé en ce qu'
on effectue l'oxydation catalytique en utilisant un catalyseur contenant du platine, sans régulation du pH.
